# EUROPÄISCHE PATENTSCHRIFT

(11) **EP 1 452 584 B1**
(45) Veröffentlichungstag und Bekanntmachung des Hinweises auf die Patenterteilung: **20.12.2006**
(21) Anmeldenummer: 04004039.6
(22) Anmeldetag: 23.02.2004
(51) Int. Cl.: C12F 3/06, A23L 2/04, A23L 1/275, A61K 31/00

(54) **Verfahren zur Gewinnung von Wertstoffen aus Trester und deren Verwendung**
Method for recovering recyclable material from dregs and use thereof
Procédé d'extraction de matériaux récyclables issue de marc et leur utilisation

(30) Priorität: 25.02.2003 DE 10307857
(43) Veröffentlichungstag der Anmeldung: 01.09.2004
(73) Patentinhaber: Universität Hohenheim, 70593 Stuttgart (DE)
(72) Erfinder: Carle, Reinhold, Prof. Dr., 72657 Altenriet (DE); Stoll, Thomas, 72649 Wolfschlugen (DE); Schweiggert, Ute, 89257 Illertissen (DE); Schieber, Andreas, Dr., 70619 Stuttgart (DE)
(74) Vertreter: Müller-Boré & Partner Patentanwälte

(56) Entgegenhaltungen:
- WO-A-02/30219
- DD-A- 254 879
- DE-A- 10 057 976
- DATABASE FSTA [Online] INTERNATIONAL FOOD INFORMATION SERVICE (IFIS), FRANFURT/MAIN, DE; DUBODEL N P ET AL: "Isolation of a natural food dye from tomato products." Database accession no. 96-1-09-j0105 XP002275011 & PISCHCHEVAYA PROMYSHLENNOST', MOSCOW 1995 MOSKOVSKAYA GOSUDARSTVENNAYA AKAD., PISHCHEVYKH PRODUKTOV, MOSCOW, RUSSIA,
- LNADBO ET AL.: "Enzyme -Assisted Extraction of Antioxidative Phenols from Black Currant Press Residues (Ribes nigrum)" JOURNAL OF AGRICULTURAL AND FOOD CHEMISTRY, Bd. 49, Nr. 7, 2001, Seiten 3169-3177, XP002275010

## Beschreibung

Die vorliegende Erfindung betrifft ein Verfahren zur Gewinnung von Wertstoffen, insbesondere oligogalacturonsäure- und carotinhaltige Wertstoffe, aus Trester, sowie deren Verwendung als Zutaten oder Zusatzstoffe in der Lebensmittel- und pharmazeutischen Industrie.

Trester (Pressrückstand) ist der vorwiegend feste Rückstand, der nach dem Auspressen des Saftes von Pflanzenbestandteilen übrig bleibt. Trester entsteht unter anderem beim Auspressen von Weintrauben, Äpfeln, Karotten oder Tomaten. Trester stellt zwar einen Reststoff dar, enthält aber noch beträchtliche Mengen an ernährungsphysiologisch interessanten Wertstoffen (Carotinoide, Polyphenole, Uronsäuren). Dies ist unter anderem darauf zurückzuführen, daß die Saftausbeute bei Obst und Gemüse, trotz Maischeenzymierung und Dekantertechnologie, lediglich ca. 60-70% beträgt, so daß rund ein Drittel der eingesetzten Rohwarenmenge als Trester zurückbleibt. Bei der Gewinnung carotinoidreicher Säfte, beispielsweise Karottensaft, bleibt im Trester bis zu 80% des Gesamtcarotins zurück, das somit bislang für die menschliche Ernährung nicht zur Verfügung stand (Sims et al. (1993), *Journal of Food Science 58,* 1129-1131). Abhängig von der Obst- oder Gemüsesorte und der Herstellungstechnologie bleiben mindestens 1,5 g Gesamtcarotinoide pro kg Trokkentrester zurück (Stoll et al. (2001) *Eurofoodchem Xl*, Norwich UK, 525-527).

Der Trester der meisten Früchte, welcher beim Entsaften anfällt, wird in erster Linie als Futtermittel, teilweise auch als Dünger verwendet. Die Nachfrage als Futtermittel ist jedoch, je nach Ernteertrag in der Landwirtschaft, starken Schwankungen unterworfen. Des weiteren ist bei der Verwendung als Futtermittel eine Trocknung des Tresters notwendig, um den mikrobiellen Verderb während der Lagerung zu vermeiden, wobei die Kosten für die Trocknung durch die Verwendung als Futtermittel kaum gedeckt werden. Außerdem stellt der Trester als Reststoff auch ein beträchtliches Umweltproblem dar und verursacht bei der Entsorgung erhebliche Kosten. Eine aus ökonomischer Sicht sinnvolle Verwertung von Trester existiert bislang nicht.

Japanischen Untersuchungen zufolge ist zwar eine Verwendung in Brot (Osawa et al. (1994), *Research Report of the Nagano State Laboratory of Food Technology 22,* 24-281994) bzw. Kuchen, Dressings und Pickles (Osawa et al. (1995), *Research Report of the Nagano State Laboratory of Food Technology 22,* 24-28) möglich, jedoch stehen einer derartigen Verwendung Vorbehalte der Verbraucher gegenüber. Henn und Kunz ((1996), *Flüssiges Obst 63*, 715-718) beschreiben die Herstellung funktioneller Drinks unter Zusatz von Karottentrester als carotinoidhaltigen Ballaststoff. Bei der sensorischen Bewertung von mit Karottentrester angereicherten handelsüblichen Multivitamingetränken wird zwar eine erhöhte Farbsättigung festgestellt, die Produkte zeichnen sich jedoch durch strohigen Geruch bzw. durch sandige Konsistenz aus.

WO 02/30219 beschreibt u.a. ein Verfahren zur Herstellung von Oligosacchariden aus Ginseng durch Verarbeitung des bei der Ginseng-Extraktion anfallenden Rückstands, welches die Behandlung einer wässrigen Suspension des Ginseng-Rückstands mit hydrolytischen Enzymen, wie Cellulase oder Pektinase, und Filtrieren des Überstands einer derartigen Suspension umfaßt. DE 100 57 976 beschreibt u.a. ein Verfahren zur Herstellung von Pektinhydrolyseprodukten unter Verwendung von pektinolytischen Enzymen. DD 254 879 beschreibt u.a. ein Verfahren zur enzymatischen Verflüssigung von roter Beete unter Verwendung von Pektinasen/Mazerasen und ggf. Cellulasen/Hemicellulasen. Der FSTA-Datenbankeintrag mit der Zugangsnummer XP-002275011 beschreibt die enzymatische Hydrolyse von beispielsweise Tomatenpaste zum Erhalt von natürlichen Lebensmittelfarbstoffen.

Der vorliegenden Erfindung liegt somit die Aufgabe zugrunde, ein Verfahren bereitzustellen, mit welchem ernährungsphysiologisch interessante Wertstoffe, wie Oligogalacturonsäuren und Carotinoide, aus Trester gewonnen werden können, sowie die Verwendung dieser aus Trester gewonnenen Wertstoffe als Zutaten oder Zusatzstoffe in Lebensmitteln und pharmazeutischen Produkten.

Diese Aufgabe wird durch die in den Ansprüchen gekennzeichneten Gegenstände gelöst.

Insbesondere wird erfindungsgemäß ein Verfahren zur Gewinnung von Wertstoffen, insbesondere oligogalacturonsäure- und carotinhaltige Wertstoffe, aus Trester bereitgestellt, welches die folgenden Schritte umfaßt:
(a) Zerkleinern des Tresters,
(b) enzymatische Hydrolyse des in Schritt (a) erhaltenen Produktes unter Einstellung des Gehalts an gesättigten und ungesättigten Oligogalacturonsäuren durch Verwendung einer Kombination aus cellulolytischen und pektinolytischen Enzymen im Verhältnis von 1:3 bis 3:1 und
(c) Passieren des in Schritt (b) erhaltenen Produktes durch eine Trennvorrichtung.

Die erfindungsgemäß hergestellten Wertstoffe bzw. Produkte bzw. Zusammensetzungen können neben Oligogalacturonsäuren und Carotinoiden weitere nutritive Inhaltsstoffe und/oder organische Mikronährstoffe enthalten.

Im erfindungsgemäßen Verfahren kann jeglicher Obst- und/oder Gemüsetrester, beispielsweise Karottentrester, verwendet werden. Das Zerkleinern des Tresters in Schritt (a) erfolgt vorzugsweise mechanisch, beispielsweise mit einer Kolloidmühle oder einem Inline Turrax. Der Schritt des Zerkleinerns kann ein- oder mehrmalig erfolgen. In einer Ausführungsform des erfindungsgemäßen Verfahrens wird der Trester unter Erhitzen, vorzugsweise bei Temperaturen bis etwa 100°C, zerkleinert.

Für die enzymatische Hydrolyse in Schritt (b) wird eine Kombination aus cellulolytischen und pektinolytischen Enzymen verwendet. Bei dieser Kombination (cellulolytische und pektinolytische Enzyme) ist das Verhältnis der Enzyme (im Bereich von 1:3 bis 3:1, bevorzugt wird ein Verhältnis im Bereich von etwa 1:1 verwendet. Durch eine bestimmte Auswahl von Enzymen kann vorteilhafterweise der Gehalt an gesättigten und/oder ungesättigten Oligogalacturonsäuren im Endprodukt eingestellt werden.

Vorzugsweise entstehen bei der enzymatischen Hydrolyse gesättigte und/oder ungesättigte Oligogalacturonsäuren mit einem Polymerisationsgrad von zwei bis zehn, beispielsweise zwei bis sieben, zwei bis sechs, zwei bis fünf, zwei bis vier oder zwei bis drei, und besonders bevorzugt mit einem Polymerisationsgrad von zwei. Der Veresterungsgrad der gesättigten und/oder ungesättigten Oligogalacturonsäuren beträgt vorzugsweise etwa 70%, beispielsweise etwa 50%, etwa 20% oder etwa 10%, und besonders bevorzugt etwa 5%. Der pH-Wert bei der enzymatischen Hydrolyse wird vorzugsweise vor der Zugabe der Enzyme bevorzugt in einem Bereich von etwa 2 bis etwa 9, beispielsweise etwa 3 bis etwa 6 oder etwa 3,5 bis etwa 4,5, und besonders bevorzugt auf einen pH-Wert von etwa 4 eingestellt. Dies kann durch die Zugabe von Genußsäuren (wie Zitronensäure, Essigsäure oder Milchsäure), Mineralsäuren oder Fruchtsaftkonzentrat erfolgen. Die enzymatische Hydrolyse wird abhängig vom Enzym oder Enzymgemisch bei einer Temperatur von etwa 5°C bis etwa 80°C, vorzugsweise von etwa 20°C bis etwa 65°C, mehr bevorzugt von etwa 45°C bis etwa 55°C und am meisten bevorzugt bei etwa 50°C durchgeführt. Die Gesamtenzymdosage bzw. der Gesamtenzymanteil der zugesetzten Enzyme sollte im erfindungsgemäßen Verfahren im Bereich von etwa 10 bis etwa 100000 ppm, vorzugsweise von etwa 500 bis etwa 5000 ppm, mehr bevorzugt von etwa 1000 bis etwa 2000 ppm und am meisten bevorzugt etwa 1500 ppm betragen. Die Dauer der enzymatischen Hydrolyse ist vom verwendeten Enzym bzw. von den verwendeten Enzymen, vom pH-Wert und der Temperatur abhängig und beträgt vorzugsweise etwa 1 bis 2 Stunden.

Das Passieren des Hydrolyseproduktes in Schritt (c) zum Entfernen von noch möglicherweise vorhandenen, nicht abgebauten, groben Partikeln, z.B. Trubpartikel, erfolgt mittels einer Trennvorrichtung, wobei gegebenenfalls abgetrennte bzw. zurückgehaltene Partikel erneut der enzymatischen Hydrolyse (Schritt (b)) zugeführt werden können. Als Trennvorrichtung kann beispielsweise ein Siebeinsatz mit Maschenweiten bis etwa 1,5 mm, bevorzugt mit Maschenweiten im Bereich von etwa 0,5 bis etwa 0,8 mm, eingesetzt werden. Durch das erneute Hydrolysieren der mittels Passieren entfernten Partikel kann vorteilhafterweise eine vollständige Reststoffverwertung erreicht werden. In einer bevorzugten Ausführungsform des erfindungsgemäßen Verfahrens, insbesondere zur Erhöhung der Ausbeute, können die Partikel mit Wasser ausgewaschen werden und das Waschwasser dem Hydrolyseprodukt zugefügt werden. Grobe Partikel sind Teilchen mit einem mittleren Partikeldurchmesser von etwa 500 µm bis etwa 1500 µm.

In einer bevorzugten Ausführungsform des erfindungsgemäßen Verfahren kann der Trester vor dem Zerkleinern, d.h. vor Schritt (a), auf eine Temperatur bis etwa 100 °C erhitzt werden. Des weiteren kann der Trester vor oder nach dem Erhitzen in Wasser oder Obst- bzw. Gemüsesaft suspendiert werden.

In einer weiteren Ausführungsform des erfindungsgemäßen Verfahrens kann nach dem Schritt des Passierens das in Schritt (c) erhaltene Produkt pasteurisiert und/oder sterilisiert werden, um eine mikrobiologische Stabilität des Produktes sicherzustellen. Ebenfalls können durch diesen Schritt die in Schritt (b) zugesetzten Enzyme inaktiviert werden. Eine Pasteurisierung ist ausreichend, wenn das Endprodukt einen pH-Wert von ≤ 4,5 besitzt, andernfalls ist eine Sterilisation erforderlich.

In einer weiteren Ausführungsform des erfindungsgemäßen Verfahrens können die im Hydrolyseschritt verwendeten Enzyme nach dem Hydrolyseschritt (b) oder in einem späteren Verfahrensschritt, beispielsweise durch die vorstehend beschriebene Pasteurisation oder Sterilisation, durch Erhitzen inaktiviert werden. Vorzugsweise wird das Hydrolyseprodukt auf beispielsweise etwa 90°C für etwa 1 bis etwa 5 Minuten erhitzt und damit die Enzyme inaktiviert.

In einer weiteren Ausführungsform des erfindungsgemäßen Verfahrens kann das in Schritt (c) erhaltene Produkt vor oder nach der Pasteurisation und/oder Sterilisation homogenisiert werden. Die Homogenisation stellt einen weiteren Schritt zur Reduzierung der Partikelgröße dar. Die Homogenisation wird bei Drücken bis etwa 450 bar, bevorzugt etwa 50 bar bis etwa 300 bar, besonders bevorzugt bei etwa 100 bar bis etwa 200 bar bei einer Temperatur von etwa 10°C bis etwa 90°C durchgeführt. Der Schritt der Homogenisation kann ein- oder mehrmalig, bevorzugt einmalig durchgeführt werden. Beim Einsatz der erfindungsgemäß hergestellten oligogalacturonsäure- und carotinhaltigen Produkte in Getränken ist der Schritt der Homogenisation jedoch entbehrlich, da beim Ausmischen von Getränken vor der Pasteurisation in der Regel eine Homogenisation erfolgt.

In einer weiteren Ausführungsform des erfindungsgemäßen Verfahrens kann nach der Homogenisation und/oder der Pasteurisation und/oder der Sterilisation eine Konzentrierung erfolgen, beispielsweise in einer Aromarückgewinnunganlage mit mehrstufigem Verdampfer, wodurch der Oligogalacturonsäure- und Carotinanteil der Zusammensetzung weiter erhöht werden kann.

Die erfindungsgemäß hergestellten oligogalacturonsäure- und carotinhaltigen Produkte können nach der Homogenisation und/oder der Pasteurisation und/oder der Sterilisation und/oder Konzentrierung beispielsweise einer Trocknung unterzogen werden, oder auf ein inertes Trägermaterial aufgebracht und anschließend durch beispielsweise Sprühtrocknung oder Gefriertrocknung getrocknet werden. Das inerte Trägermaterial kann beispielsweise Maltodextrin oder hochdisperse Kieselsäure umfassen.

Die erfindungsgemäß hergestellten oligogalacturonsäure- und carotinhaltigen Produkte können in getrockneter oder viskoser Form als färbende Zusatzstoffe oder Zutaten für Lebensmittel, vorzugsweise in trockenen Produkten, wie beispielsweise Trockensuppen, in festen Produkten, wie beispielsweise Margarine und Nudeln, oder in flüssigen Produkten, wie beispielsweise Getränke, verwendet werden. Des weiteren können die oligogalacturonsäure- und carotinhaltigen Produkte als funktionelle Bestandteile in Lebensmitteln, insbesondere als Provitamin A-Quelle, verwendet werden. Darüber hinaus können die erfindungsgemäß hergestellten oligogalacturonsäure- und carotinhaltigen Produkte als Bestandteile in pharmazeutischen Zusammensetzungen enthalten sein.

Ferner wird ein pharmazeutisches Produkt, welches die nach dem erfindungsgemäßen Verfahren hergestellten Wertstoffe als pharmakologisch aktiven Bestandteil enthält, bereitgestellt.

Durch das erfindungsgemäße Verfahren wird erstmalig eine im wesentlichen vollständige Verwertung von Trester unter Gewinnung von vorzugsweise oligogalacturonsäure- und carotinhaltigen Produkten ermöglicht. Darüber hinaus ist es mit dem erfindungsgemäßen Verfahren möglich, den Gehalt an gesättigten und/oder ungesättigten Oligogalacturonsäuren durch die Auswahl geeigneter Enzyme einzustellen. Gesättigte und/oder ungesättigte Oligogalacturonsäuren bestehen vorwiegend aus (1→4)-verknüpfter α-D-Galacturonsäure, wobei ungesättigte Oligogalacturonsäuren eine terminale Uronsäureeinheit, vorzugsweise α-D-Galacturonsäure, am nichtreduzierenden Ende enthalten, welche eine Doppelbindung besitzt, die insbesondere zwischen dem C₄- und C₅-Atom lokalisiert ist. Oligogalacturonsäuren mit einem Polymerisationsgrad von zwei bis drei werden aufgrund ihrer Adhäsion an pathogene

Keime zur Therapie infektiöser Diarrhöen vorgeschlagen.

Die folgenden Beispiele erläutern die vorliegende Erfindung näher, ohne sie einzuschränken.

### BEISPIELE

### Beispiel 1: Gewinnung von oligogalacturonsäure- und carotinhaltigen Wertstoffen aus Trester

1 Teil Karottennaßtrester (2 kg; 274 mg Gesamtcarotin/kg) wird in 5 Teilen Wasser suspendiert und der suspendierte Trester mittels der Kolloidmühle mechanisch zerkleinert. Anschließend wird eine enzymatische Hydrolyse mit den Enzymen Pectinex Ultra SP-L (Novo Nordisk, Dittingen, Schweiz) und Cytolase CL (DSM Food Specialities, Lille Cedex, Frankreich) im Verhältnis 1:1 mit einer Gesamtmenge an aktiven Enzymen von 1500 ppm bei 50°C und pH 4,0 eingesetzt, wobei die Dauer der enzymatische Hydrolyse eine Stunde beträgt. Zur Inaktivierung der Enzyme wird die Suspension für eine Minute auf 90°C erhitzt und anschließend abgekühlt. Durch Einsatz einer Passiermaschine (0,5 mm Maschenweite) werden nicht abgebaute grobe Partikel abgetrennt, welche wiederum der enzymatischen Hydrolyse zugeführt werden. Die Homogenisation erfolgt bei 180 bar. Abschließend wird das Hydrolysat in einer Aromarückgewinnungsanlage aufkonzentriert. Als Endprodukt wird ein oligogalacturonsäure- und carotinangereichertes Produkt mit einem Gesamtcarotingehalt von 64 mg pro kg Endprodukt und einem Gehalt an gesättigten Oligogalacturonsäuren (Mono-, Di- und Trimer) von 1087,8 mg/L (siehe Tabelle 1) erhalten. Die Bestimmung des Carotingehalts erfolgt nach der Methode von Stoll et al. (2003, *Food, Agriculture & Environment* 1, 88-92). Ein signifikanter Abbau der Carotinoide ist während des gesamten Prozesses nicht zu beobachten.

### Beispiel 2: Enzymatische Hydrolyse zur Ausbeutesteigerung von Oligogalacturonsäuren mit einem Polymerisationsgrad von zwei und drei

Dieses Beispiel wird wie Beispiel 1 ausgeführt, jedoch wird zur enzymatischen Hydrolyse 1770 ppm Rohapect MA plus (Röhm Enzyme, Darmstadt) und 1640 ppm Cytolase CL bei 50°C und pH 4 eingesetzt, wobei die Dauer der enzymatischen Hydrolyse 2 Stunden beträgt. Zur Inaktivierung der Enzyme wird die Suspension für 5 Minuten auf 90°C erhitzt und anschließend abgekühlt. Die Gehalte an gesättigten Oligogalacturonsäuren im Endprodukt sind in Tabelle 1 aufgelistet.

### Beispiel 3: Identifizierung und Quantifizierung gesättigter Oligogalacturonsäuren mittels Flüssigkeitschromatographie-Massenspektrometrie (LC-MS)

Die Identifizierung gesättigter Oligogalacturonsäuren wird mittels LC-MS nach einer Methode von Stoll et al. (2002, *Carbohydrate Research 337*, 2481-2486) durchgeführt. Die Quantifizierung erfolgt mittels LC-MS im SIM-Modus (selected ion monitoring). Standard-Kalibriergeraden der Mono-, Di- und Trigalacturonsäure werden im Konzentrationsbereich 5 bis 500 mg/L erstellt und die Gehalte an Oligogalacturonsäuren in der Probe mittels linearer Regression ermittelt (siehe Tabelle 1).

### Beispiel 4: Einsatz des oligogalacturonsäure- und carotinhaltigen Produktes in einem Modellgetränk

21 kg Modellgetränk wird aus 50% Fruchsaftgehalt, 12° Brix, 450mg/L Ascorbinsäure, 12mg/L Provitamin A und 0,05% Pektin gemischt. Hierzu werden 10,498 kg natürtrüber Apfelsaft und 3,762 kg oligogalacturonsäure- und carotinhaltigen Produkt eingemischt, entlüftet, homogenisiert und erneut entlüftet. Die Bestimmung des Carotingehalts erfolgt nach der Methode von Stoll et al. (2003, *Food, Agriculture & Environment* 1, 88-92). Anschließend werden 1,629 kg Saccharoselösung (68° Brix), Ascorbinsäure (430 mg/kg), Citronensäure (0,21%) und 1,050 kg Pektinlösung (1%, m/m) zugegeben und mit Wasser aufgefüllt. Das Modellgetränk wird mittels Röhrenerhitzer für 60 s bei 90°C pasteurisiert, in 0,5 L Flaschen abgefüllt und unter Heißdampfinjektion verschlossen.

**Tabelle 1:**

| | Beispiel 1 (Konzentration mg/L) | Beispiel 2 (Konzentration mg/L) |
|---|---|---|
| **DP 1,** Mittelwert | 972,1 | 527,3 |
| **DP 2,** Mittelwert | 73,5 | 666,5 |
| **DP 3,** Mittelwert | 42,2 | 1322,8 |
| **DP 4,** Mittelwert | 2,3 | 75,9 |
| **DP 3-MeOH,** Mittelwert | 35,4 | 269,4 |
| **DP 4-MeOH,** Mittelwert | 18,8 | 13,1 |
| Summe **DP 1** bis **DP-3** | 1087,8 | 2516,6 |
| Gesamtsumme **DP** | 1144,3 | 2875,0 |

| | | |
|---|---|---|
| DP: Polymerisationsgrad DP 3: Polymerisationsgrad von drei (Trimer) DP 3-MeOH: Polymerisationsgrad von drei (Trimer) mit einer Methylgruppe | | |

Außerdem wurden in Beispiel 1 folgende gesättigte Oligogalacturonsäuren noch qualitativ nachgewiesen: DP 4-2MeOH, DP 5-2MeOH, DP 5-3MeOH, DP 6-2MeOH, DP 6-3MeOH, DP 7-3MeOH (Polymerisationsgrad von sieben (Heptamer) mit drei Methylgruppen), DP 7-4MeOH, DP 8-4MeOH, DP 8-5MeOH, DP 9-5MeOH, DP 9-6MeOH, DP 10-6MeOH

## Patentansprüche

1. Verfahren zur Gewinnung von Oligogalacturonsäure- und Carotin-haltigen Wertstoffen aus Trester, umfassend die Schritte:
(a) Zerkleinern des Tresters,
(b) enzymatische Hydrolyse des in Schritt (a) erhaltenen Produktes unter Einstellung des Gehalts an gesättigten und ungesättigten Oligogalacturonsäuren durch Verwendung einer Kombination aus cellulolytischen und pektinolytischen Enzymen im Verhältnis von 1:3 bis 3:1 und
(c) Passieren des in Schritt (b) erhaltenen Produkts durch eine Trennvorrichtung.

2. Verfahren nach Anspruch 1, wobei die beim Passieren abgetrennten Partikel erneut der enzymatische Hydrolyse zugeführt werden.

3. Verfahren nach Anspruch 1 oder 2, wobei der Trester vor dem Zerkleinern erhitzt wird.

4. Verfahren nach einem der vorhergehenden Ansprüche, wobei der Trester vor dem Zerkleinern in Wasser oder Saft suspendiert wird.

5. Verfahren nach einem der vorhergehenden Ansprüche, wobei das in Schritt (c) erhaltene Produkt in einem weiteren Schritt pasteurisiert und/oder sterilisiert wird.

6. Verfahren nach einem der vorhergehenden Ansprüche, wobei das in Schritt (c) erhaltene Produkt vor oder nach dem Schritt der Pasteurisation und/oder Sterilisation homogenisiert wird.

7. Verfahren nach Anspruch 5 oder 6, wobei das homogenisierte und/oder pasteurisierte und/oder sterilisierte Produkt getrocknet wird oder auf ein inertes Trägermaterial aufgebracht wird.

8. Verfahren nach einem der vorhergehenden Ansprüche, wobei das in Schritt (b) verwendete Enzymgemisch inaktiviert wird.

9. Verfahren nach einem der vorhergehenden Ansprüche, wobei die enzymatische Hydrolyse bei einer Temperatur von etwa 5°C bis etwa 80°C durchgeführt wird.

10. Verfahren nach einem der vorhergehenden Ansprüche, wobei die enzymatische Hydrolyse bei einem pH-Wert von etwa 1 bis etwa 9 durchgeführt wird.

11. Verfahren nach einem der vorhergehenden Ansprüche, wobei die zum Passieren eingesetzte Trennvorrichtung Siebeinsätze mit Maschenweiten bis etwa 1,5 mm aufweist.

12. Verfahren nach einem der vorhergehenden Ansprüche, wobei die Homogenisierung bei einem Druck bis zu etwa 450 bar durchgeführt wird.

13. Oligogalacturonsäure- und carotinhaltige Wertstoffe, erhältlich durch ein Verfahren nach einem der Ansprüche 1 bis 12.

14. Verwendung der nach einem der Ansprüche 1 bis 12 hergestellten Wertstoffe als Zutat oder färbender Zusatzstoff und/oder funktioneller Bestandteil in Lebensmitteln oder als Zusatzstoff in pharmazeutischen Produkten.

15. Pharmazeutisches Produkt, enthaltend die nach einem der Ansprüche 1 bis 12 hergestellten Wertstoffe als pharmakologisch aktiven Bestandteil.

## Claims

1. Method for the production of oligogalacturonic acid-containing and carotene-containing recyclable materials from pomace comprising the steps:
(a) comminution of the pomace,
(b) enzymatic hydrolysis of the product obtained in step (a) with the content of saturated and unsaturated oligogalacturonic acids being set by using a combination of cellulolytic and pectinolytic enzymes in the ratio of 1:3 to 3:1 and
(c) passage of the product obtained in step (b) through a separation apparatus.

2. Method according to Claim 1, the particles separated off in the course of the passage being fed back to the enzymatic hydrolysis.

3. Method according to Claim 1 or 2, the pomace being heated before the comminution.

4. Method according to one of the preceding claims, the pomace being suspended in water or juice before the comminution.

5. Method according to one of the preceding claims, the product obtained in step (c) being pasteurized and/or sterilized in a further step.

6. Method according to one of the preceding claims, the product obtained in step (c) being homogenized before or after the pasteurization and/or sterilization step.

7. Method according to Claim 5 or 6, the homogenized and/or pasteurized and/or sterilized product being dried or applied to an inert carrier material.

8. Method according to one of the preceding claims, the enzyme mixture used in step (b) being inactivated.

9. Method according to one of the preceding claims, the enzymatic hydrolysis being carried out at a temperature of about 5°C to about 80°C.

10. Method according to one of the preceding claims, the enzymatic hydrolysis being carried out at a pH of about 1 to about 9.

11. Method according to one of the preceding claims, the separation apparatus used for the passage having sieve inserts of mesh widths up to about 1.5 mm.

12. Method according to one of the preceding claims, the homogenization being carried out at a pressure up to about 450 bar.

13. Oligogalacturonic acid-containing and carotene-containing recyclable materials obtainable by a method according to one of Claims 1 to 12.

14. Use of the recyclable materials produced according to one of Claims 1 to 12 as ingredient or colouring additive and/or functional constituent in foods or as additive in pharmaceutical products.

15. Pharmaceutical product containing the recyclable materials produced according to one of Claims 1 to 12 as pharmacologically active constituent.

## Revendications

1. Procédé d'extraction de matériaux recyclables contenant des acides oligogalacturoniques et du carotène à partir de marc de raisin, comprenant les étapes de :
(a) pulvérisation du marc,
(b) hydrolyse enzymatique du produit obtenu à l'étape (a) pour extraction du contenu en acides oligogalacturoniques saturés et insaturés par utilisation d'une combinaison d'enzymes cellulolytiques et pectinolytiques dans un rapport entre 1:3 et 3:1, et
(c) passage du produit obtenu à l'étape (b) par un dispositif de séparation.

2. Procédé selon la revendication 1 dans lequel les particules séparées lors du passage sont réintroduites dans l'hydrolyse enzymatique.

3. Procédé selon la revendication 1 ou 2 dans lequel le marc est chauffé avant la pulvérisation.

4. Procédé selon une quelconque des revendications précédentes, dans lequel le marc est mis en suspension dans l'eau ou dans un jus avant la pulvérisation.

5. Procédé selon une quelconque des revendications précédentes, dans lequel le produit obtenu à l'étape (c) est pasteurisé et/ou stérilisé dans une étape supplémentaire.

6. Procédé selon une quelconque des revendications précédentes, dans lequel le produit obtenu à l'étape (c) est homogénéisé avant ou après l'étape de pasteurisation et/ou de stérilisation.

7. Procédé selon la revendication 5 ou 6 dans lequel le produit homogénéisé et/ou pasteurisé et/ou stérilisé est séché ou appliqué sur un matériau support inerte.

8. Procédé selon une quelconque des revendications précédentes, dans lequel le mélange enzymatique utilisé à l'étape (b) est inactivé.

9. Procédé selon une quelconque des revendications précédentes, dans lequel l'hydrolyse enzymatique est réalisée à une température entre environ 5°C et environ 80°C.

10. Procédé selon une quelconque des revendications précédentes, dans lequel l'hydrolyse enzymatique est réalisée à un pH dont la valeur est comprise entre environ 1 et 9.

11. Procédé selon une quelconque des revendications précédentes, dans lequel le dispositif de séparation utilisé lors du passage présente des dispositifs de tamisage avec des ouvertures de maille d'environ 1,5 mm au maximum.

12. Procédé selon une quelconque des revendications précédentes, dans lequel l'homogénéisation est effectuée sous une pression d'environ 450 bar au maximum.

13. Matériaux recyclables contenant des acides oligogalacturoniques et du carotène disponibles à partir d'un procédé selon une quelconque des revendications 1 à 12.

14. Utilisation des matériaux recyclables produits selon une quelconque des revendications 1 à 12 comme ingrédient ou comme additif colorant et/ou comme composant fonctionnel dans des produits agroalimentaires ou comme additif dans des produits pharmaceutiques.

15. Produit pharmaceutique contenant les matériaux recyclables produits selon une quelconque des revendications 1 à 12 comme composant pharmacologiquement actif.
